# EUROPEAN PATENT APPLICATION

(11) **EP 4 656 281 A1**
(43) Date of publication of application: **03.12.2025**
(21) Application number: 23918533.3
(22) Date of filing: 14.11.2023
(51) Int. Cl.: B01J 8/06, C07B 61/00, C07C 29/152, C07C 31/04

(54) **CHEMICAL REACTION DEVICE AND CHEMICAL REACTION METHOD**

(30) Priority: 23.01.2023 JP 2023008258
(71) Applicant: Sumitomo Chemical Company, Limited, Tokyo 103-6020 (JP)
(72) Inventor: SUZUTA, Tetsuya, Niihama-shi, Ehime 792-8521 (JP); SATO, Yuichi, Niihama-shi, Ehime 792-8521 (JP); TASHIRO, Yuya, Niihama-shi, Ehime 792-8521 (JP); YAEGASHI, Yuta, Niihama-shi, Ehime 792-8521 (JP)
(74) Representative: Winter, Brandl - Partnerschaft mbB
(86) International application number: PCT/JP2023/040854
(87) International publication number: WO 2024/157578

(57) **Abstract**

Thermal efficiency is improved. This chemical reaction device includes: a catalyst layer (3); a first heat exchange section (52), a surface temperature of which is maintained not higher than a dew point of a reacted gas (32) produced by a reaction; a dividing wall (60); and a flow path forming section (70) located within a space (7) and forming a flow path for the reacted gas (32) that flows from the catalyst layer (3) into the space (7) through an opening (61). The flow path forming section (70) includes a baffle (71) that enables heat exchange in the flow path between a portion of the reacted gas (32) which portion flows from the catalyst layer (3) toward the first heat exchange section (52) and a portion of the reacted gas (32) which portion flows from the first heat exchange section (52) toward the catalyst layer (3).

## Description

### Technical Field

The present invention relates to a chemical reaction device and a chemical reaction method each causing a chemical reaction for obtaining a product from a raw material gas to proceed in a gaseous phase with use of a catalyst.

### Background Art

Patent Literature 1 discloses a chemical reaction device for carrying out a reaction, a product of which contains a component having a boiling point higher than that of a main component of a raw material gas, and progress of which in a gaseous phase is restricted by a chemical equilibrium between a raw material and the product. The chemical reaction device, which is a so-called internal condensation type reaction device, causes the product to be condensed in the device and collects the condensed product out of a reaction container, so that the chemical equilibrium is shifted toward the product and thus the reaction proceeds.

### Citation List

### [Patent Literature]

[Patent Literature 1]
PCT International Publication No. WO 2021/060145 A1

### Summary of Invention

### Technical Problem

In a reaction method using a reaction device such as that disclosed in Patent Literature 1, it is desired to improve thermal efficiency.

It is an object of an aspect of the present invention to improve thermal efficiency of a reaction carried out with use of an internal condensation type reaction device.

### Solution to Problem

In order to attain the object, a chemical reaction device in accordance with an aspect of the present invention is a chemical reaction device that causes a reaction to proceed, a product of the reaction containing a component having a boiling point higher than that of a raw material gas, progress of the reaction in a gaseous phase being restricted by a chemical equilibrium between a raw material and the product, the chemical reaction device including: a catalyst layer to which the raw material gas is supplied and which is filled with a catalyst that promotes the reaction; a first heat exchange section provided apart from the catalyst layer with a space between the first heat exchange section and the catalyst layer, a temperature of a surface of the first heat exchange section on a space side being maintained not higher than a dew point of a reacted gas produced by the reaction; a dividing wall located between the catalyst layer and the space and having an opening through which the reacted gas passes; and a flow path forming section located within the space and forming a flow path for the reacted gas that flows from the catalyst layer into the space through the opening, the flow path forming section includes a baffle that enables heat exchange in the flow path between a portion of the reacted gas which portion flows from the catalyst layer toward the first heat exchange section and a portion of the reacted gas which portion flows from the first heat exchange section toward the catalyst layer.

### Advantageous Effects of Invention

An aspect of the present invention makes it possible to improve thermal efficiency of a reaction carried out with use of an internal condensation type reaction device.

### Brief Description of Drawings

Fig. 1 is a cross-sectional view of a reaction device in accordance with Embodiment 1, the cross-sectional view being taken along a plane perpendicular to a bottom surface of the reaction device.
Fig. 2 is an enlarged view of a region R1 in Fig. 1.
Fig. 3 is a cross-sectional view taken along line III-III in Fig. 2.
Fig. 4 is an enlarged view of a part of a variation of the above reaction device.
Fig. 5 is a cross-sectional view taken along line V-V in Fig. 4.
Fig. 6 is a cross-sectional view of a main part of a variation of the above reaction device.
Fig. 7 is a cross-sectional view of a reaction device in accordance with Embodiment 2, the cross-sectional view being taken along a plane perpendicular to a bottom surface of the reaction device.
Fig. 8 is an enlarged view of a region R2 in Fig. 7.
Fig. 9 is a cross-sectional view of a reaction device in accordance with Embodiment 3, the cross-sectional view being taken along a plane perpendicular to a bottom surface of the reaction device.
Fig. 10 is an enlarged view of a region R3 in Fig. 9.
Fig. 11 is a view schematically illustrating a configuration of a model device used for a simulation of Example 1.
Fig. 12 is a view schematically illustrating a configuration of a model device used for a simulation of Example 2.
Fig. 13 is a view schematically illustrating a configuration of a model device used for a simulation of Comparative Example.

### Description of Embodiments

### Embodiment 1

### (Configuration of reaction device 100)

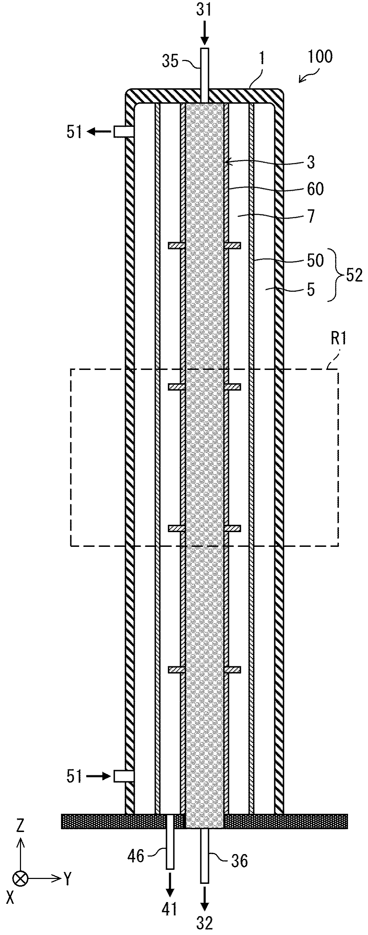

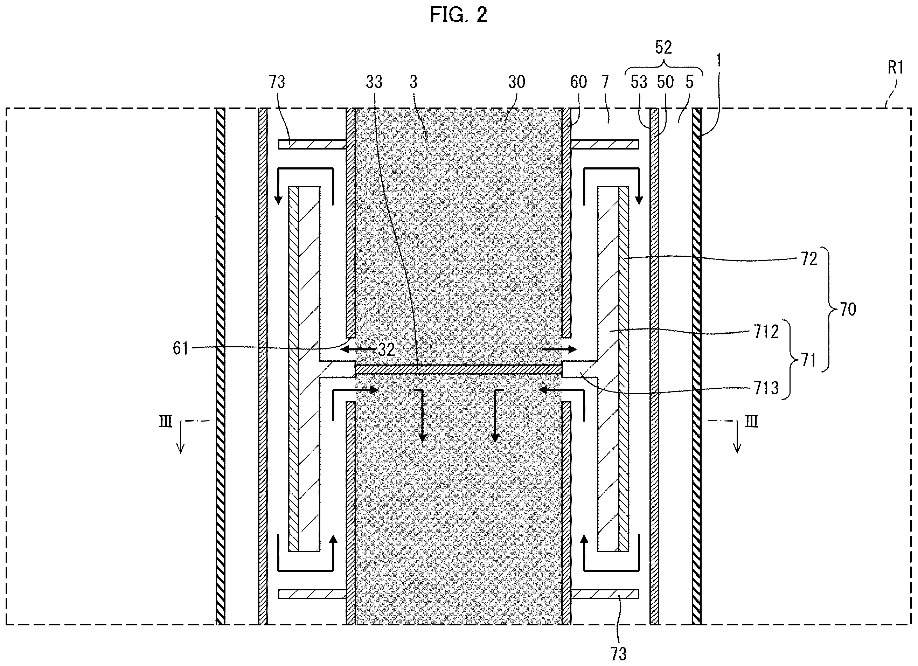

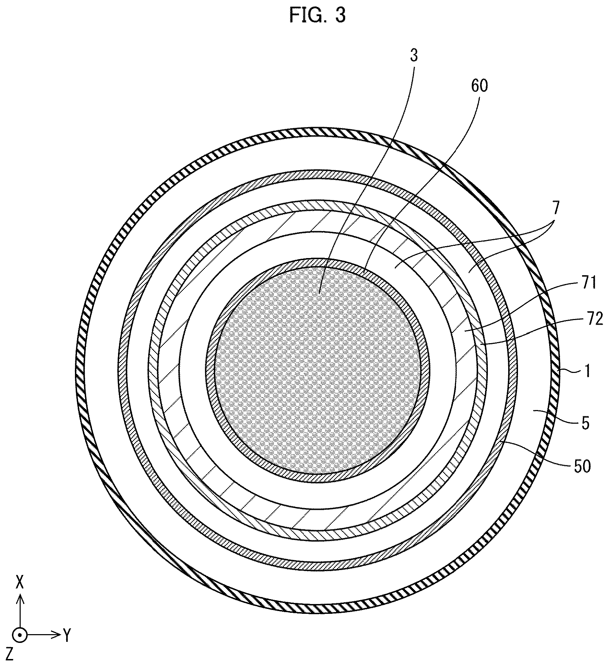

The following description will discuss an embodiment of the present invention in detail. Fig. 1 is a cross-sectional view of a reaction device 100 (chemical reaction device) in accordance with Embodiment 1, the cross-sectional view being taken along a plane perpendicular to a bottom surface of the reaction device 100. Fig. 2 is an enlarged view of a region R1 in Fig. 1. Fig. 3 is a cross-sectional view taken along line III-III in Fig. 2. The cross-sectional view is an end view showing only a structure of an end surface in the cross section. Note that the X-axis, the Y-axis, and the Z-axis in the drawings define directions in a three-dimensional space in each of the drawings. In the present specification, the Z-axis is a direction perpendicular to an installation surface of the reaction device 100.

The reaction device 100 is a chemical reaction device that causes a reaction, a product of which contains a component having a boiling point higher than that of a main component of a raw material gas 31, and progress of which in a gaseous phase is restricted by a chemical equilibrium between a raw material and the product, to proceed.

In the reaction device 100, the product is condensed and collected out of a reaction container. This causes the chemical equilibrium to be shifted toward the product and thus enables the reaction to proceed. The reaction device 100 can be suitably used as a device for carrying out a chemical reaction in which the above reaction is an exothermic reaction. In particular, the reaction device 100 can be suitably used as a device for carrying out chemical reactions which are represented by the respective following formulae (1) to (3), in which the raw material gas 31 contains a carbon oxide and hydrogen, and the product of which contains methanol.

(1): CO+2H₂ 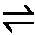 CH₃OH

(2): CO₂+3H₂ 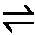 CH₃OH+H₂O

(3): CO₂+H₂ 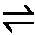 CO+H₂O

The symbol " 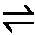 " in the above reaction formulae each indicate that this reaction is an equilibrium reaction. The methanol synthesis reaction in each of the above formulae (1) to (3) is an exothermic reaction.

Further, the reaction device 100 can be used also for carrying out a reaction that yields dimethyl ether or ammonia as a product.

As illustrated in Figs. 1 and 2, the reaction device 100 includes a reaction container 1. The reaction container 1 has a cylindrical body and, for example, is a metallic container made of pressure-resistant stainless steel. The reaction device 100 includes a cylindrical catalyst layer 3 located at a center of the reaction container 1, a first heat exchange section 52 provided with a space 7 interposed between the first heat exchange section 52 and the catalyst layer 3, a dividing wall 60 located between the catalyst layer 3 and the space 7, and a flow path forming section 70 located within the space 7.

The dividing wall 60 has an opening 61 through which a reacted gas 32 produced by a reaction in the catalyst layer 3 passes. The flow path forming section 70 forms in the space 7 a flow path for the reacted gas 32 that flows from the catalyst layer 3 into the space 7 through the opening 61.

Before describing the configuration of the reaction device 100 in detail, the following will first describe a reaction flow in the reaction device 100. In the present embodiment, the description will be given on the assumption that the reaction that occurs in the catalyst layer 3 is an exothermic reaction.

The raw material gas 31 is supplied through a raw material gas inlet 35 formed at an upper part of the catalyst layer 3, and comes into contact with the catalyst 30 filling the catalyst layer 3, so that a reaction proceeds. The direction in which the raw material gas 31 is supplied is a Z-axis negative direction. The reacted gas 32 produced by the reaction passes through the opening 61 in the dividing wall 60 into the space 7. In Fig. 2, the direction in which the reacted gas 32 travels is indicated by an arrow. The reacted gas 32 that has flowed into the space 7 travels along the flow path formed by the flow path forming section 70. In the flow path, at least part of a product contained in the reacted gas 32 cooled by the first heat exchange section 52 down to a temperature not higher than a dew point of the reacted gas 32 is condensed. The product that has been condensed and liquefied is collected as a condensate 41 through a condensate collection opening 46.

The reacted gas 32 flowing into the space 7 from a catalyst layer 3 side also contains an unreacted raw material gas. However, a main component contained in the unreacted raw material gas is not condensed at the first heat exchange section 52. The unreacted raw material gas and a remaining reacted gas 32, which remains after the at least part of the product is condensed, return to the catalyst layer 3 by traveling along the flow path formed by the flow path forming section 70.

The reacted gas 32 containing the raw material which has not been reacted in the catalyst layer 3 is collected through a reacted gas collection opening 36.

A detailed configuration of each part of the reaction device 100 is described below.

The catalyst layer 3 is filled with the catalyst 30 that promotes a reaction. The catalyst layer 3 is a region in which, upon supply of the raw material gas 31 into the catalyst layer 3, the raw material gas 31 and the catalyst 30 come into contact with each other, so that a reaction proceeds. The catalyst 30 can be, for example, a catalyst containing copper and zinc oxide as main components. A part that is an upstream part of the catalyst layer 3 and that is between the catalyst layer 3 and the reaction container 1 may be filled with any filling (not illustrated). Furthermore, in order to support the catalyst 30, the catalyst layer 3 may have a bottom part that is provided with, for example, a supporting plate (not illustrated) made of a porous member.

The dividing wall 60 is a wall that separates the catalyst layer 3 and the space 7 from each other. The dividing wall 60 has a cylindrical shape concentric with the reaction container 1, and may be made of, for example, carbon steel or stainless steel such as SUS304. The dividing wall 60 has the opening 61 through which the reacted gas passes. The shape of the opening 61 is not particularly limited, but may be circular or rectangular. The dividing wall 60 may have, for example, a plurality of openings 61 along a periphery of the dividing wall 60 at a given height position.

In a case where an opening diameter (opening width) of the opening 61 is large in comparison to the catalyst 30, the opening 61 may be provided with, for example, a porous member that does not allow the catalyst 30 to pass through but allows the reacted gas 32 to pass through. This makes it possible to reduce the likelihood that the catalyst 30 flows into the space 7.

The first heat exchange section 52 is a heat exchanger whose surface temperature on a space 7 side is maintained not higher than the dew point of the reacted gas 32. The first heat exchange section 52 cools the reacted gas 32 by heat exchange between the reacted gas 32 and a first heat transfer medium 51 which passes through the first heat exchange section 52. The first heat exchange section 52 includes a first heat exchange wall 50 and a first heat transfer medium region 5 located on a side of the first heat exchange wall 50 which side faces away from the space 7. The first heat exchange wall 50 has a cylindrical shape concentric with the reaction container 1 and is constituted by a member through which a fluid cannot pass. The first heat exchange wall 50 may be made of, for example, carbon steel or stainless steel such as SUS304. The first heat transfer medium region 5 is a region through which the first heat transfer medium 51 flows. In the present embodiment, the first heat transfer medium region 5 is a region that exists between the first heat exchange wall 50 and the reaction container 1.

The first heat exchange wall 50 has a cooling surface 53 on a space 7 side. The cooling surface 53 is maintained at a temperature not higher than the dew point of the reacted gas 32, so that the reacted gas 32 in the space 7 is cooled. As a result, at least part of the product contained in the reacted gas 32 is condensed on the surface of the cooling surface 53 and in the space 7.

Note that the wording "dew point of the reacted gas 32" means a temperature at which condensation starts in a case where the reacted gas 32 is cooled in a state where a reaction in a gaseous phase has reached a chemical equilibrium at a temperature and a pressure at which the reacted gas 32 is present in the catalyst layer 3.

When a composition of the gaseous phase and the pressure are given, the dew point of the reacted gas 32 can be calculated simultaneously with a composition of a condensate by carrying out an appropriate vapor-liquid equilibrium calculation with use of a vapor-liquid equilibrium model. In a case where the pressure is a high pressure exceeding 1 MPa, the vapor-liquid equilibrium model can be, for example, an extended cubic equation of state such as the Peng-Robinson equation or the Redich-Kwong-Soave equation.

The first heat exchange section 52 can maintain the cooling surface 53 at a temperature not higher than the dew point of the reacted gas 32 by allowing the first heat transfer medium 51 to flow through the first heat transfer medium region 5. In a case where the product is methanol, examples of the first heat transfer medium 51 include liquid or boiling liquid at -20°C to 150°C (e.g. water, methanol water, ethylene glycol water, ammonia, ammonia water, a hydrocarbon compound such as pentane, and a chlorofluorocarbon compound such as 1,1,1,3,3-pentafluoropropane).

The flow path forming section 70 is located within the space 7 and forms a flow path for the reacted gas 32 that has flowed from the catalyst layer 3 into the space 7. The flow path forming section 70 includes a baffle 71. The baffle 71 forms a flow path for the reacted gas 32 and enables heat exchange between a portion of the reacted gas 32 which portion flows from the catalyst layer 3 toward the first heat exchange section 52 and a portion of the reacted gas 32 which portion flows from the first heat exchange section 52 toward the catalyst layer 3.

In the present embodiment, the baffle 71 includes a cylinder 712 which extends in the Z-axis direction and a disk 713 which is joined to an inner wall of the cylinder 712. An inner diameter of the cylinder 712 is more than an outer diameter of the dividing wall 60, and an outer diameter of the cylinder 712 is less than an inner diameter of the first heat exchange section 52 (first heat exchange wall 50). The cylinder 712 may have a thickness in a range of 1/15 to 1/1.2 of a distance between the dividing wall 60 and the first heat exchange section 52.

The disk 713 is a plate-like member having a doughnut shape and connects the inner wall of the cylinder 712 to the dividing wall 60 (or to a first partition plate 33, which will be described later). Specifically, an outer edge (outer side surface) of the disk 713 is connected to the cylinder 712, and an inner edge (inner side surface) of the disk 713 is joined to a portion of the dividing wall 60 which portion is located between the plurality of openings 61 provided at a given height position. Alternatively, the inner edge of the disk 713 may be joined to the first partition plate 33 described later. Note that a diameter (a length in the Z-axis direction) of each opening 61 is greater than a thickness of the disk 713.

Because the disk 713 is joined to the dividing wall 60 between the openings 61, the openings 61 are each divided by the disk 713. A portion of each opening 61 which portion is located upstream of the disk 713 functions as an inflow port through which the reacted gas 32 flows from the catalyst layer 3 into the space 7. A portion of each opening 61 which portion is located downstream of the disk 713 functions as an outflow port through which the reacted gas 32 flows from the space 7 into the catalyst layer 3. By having the above configuration, the flow path forming section 70 forms a flow path for the reacted gas 32 that has flowed from the catalyst layer 3 into the space 7. Since the reaction device 100 has the flow path forming section 70, the reacted gas 32 that has flowed from the catalyst layer 3 into the space 7 travels in the space 7 while changing a traveling direction a plurality of times. Specifically, the reacted gas 32 that has flowed from the catalyst layer 3 into the space 7 through the inflow port travels in the space 7 while changing a traveling direction a plurality of times along a flow path defined by the dividing wall 60, the baffle 71, and a second partition plate 73 (described later) that is located above and below the baffle 71.

The baffle 71 is made of a material having a high thermal conductivity. For example, the baffle 71 may contain a material having a thermal conductivity higher than that of the dividing wall 60. More specifically, the baffle 71 may contain, for example, at least one material selected from the group consisting of copper, aluminum, and carbon. Due to the use of the material having a high thermal conductivity, the baffle 71 enables heat exchange through the baffle 71. More specifically, the baffle 71 enables heat exchange between a portion of the reacted gas 32 which portion flows from the catalyst layer 3 toward the first heat exchange section 52 and a portion of the reacted gas 32 which portion flows from the first heat exchange section 52 toward the catalyst layer 3 in the flow path of the reacted gas 32 in the space 7. In other words, the baffle 71 enables heat exchange between a high-temperature portion of the reacted gas 32, which has been heated by a reaction heat of the reaction in the catalyst layer 3, and a low-temperature portion of the reacted gas 32, which has been cooled by the first heat exchange section 52.

By the above heat exchange, the high-temperature portion of the reacted gas 32, which has flowed into the space 7 from the catalyst layer 3 is cooled by heat exchange with the low-temperature portion of the reacted gas 32 and travels toward the first heat exchange section 52. That is, after flowing into the space 7, the high-temperature portion of the reacted gas 32 is pre-cooled and travels toward the first heat exchange section 52. The low-temperature portion of the reacted gas 32, which has been cooled by the first heat exchange section 52, is heated by heat exchange with the high-temperature portion of the reacted gas and travels toward the catalyst layer 3. That is, after being cooled at the first heat exchange section 52, the low-temperature portion of the reacted gas 32 is preheated and returns to the catalyst layer 3. The heat exchange thus carried out in the space 7 improves the thermal efficiency of the reaction device 100.

The flow path forming section 70 may include a heat insulating member 72 located between the baffle 71 and the first heat exchange section 52. For example, as illustrated in Fig. 2, the heat insulating member 72 may be provided on a surface of the baffle 71 which surface faces the first heat exchange section 52. The heat insulating member 72 may be made of a material such as wool-like, sheet-like or paint-like ceramic, cellulose fiber, sheep's wool, carbonized cork, polystyrene foam, urethane foam or phenol foam. This makes it possible to reduce an effect of heat from the first heat exchange section 52 on the baffle 71 and further improve the efficiency of heat exchange by the baffle 71.

There may be a plurality of flow path forming sections 70 arranged along the Z axis. In a case where the plurality of flow path forming sections 70 are arranged, the flow path forming sections 70 are separated from each other by the second partition plate 73. The second partition plate 73 enables heat exchange between gas passing on a side of an upper surface of the second partition plate 73 and gas passing on a side of a lower surface of the second partition plate 73.

The second partition plate 73 is a plate-like member having a doughnut shape. An inner edge (inner side surface) of the second partition plate 73 is joined to the dividing wall 60. Between an outer side surface of the second partition plate 73 and the first heat exchange section 52, a gap or a hole is formed through which a liquefied product produced by cooling and condensing of the reacted gas can pass. The second partition plate 73 has a high thermal conductivity. The second partition plate 73 may be made of the same material as the baffle 71. For example, the second partition plate 73 may contain at least one material selected from the group consisting of copper, aluminum, and carbon.

Due to the use of the material having a high thermal conductivity, the second partition plate 73 enables heat exchange through the second partition plate 73. More specifically, the second partition plate 73 enables heat exchange between a high-temperature portion of the reacted gas 32, which has been heated by a reaction heat of the reaction in the catalyst layer 3, and a low-temperature portion of the reacted gas 32, which has been cooled by the first heat exchange section 52. The high-temperature portion of the reacted gas 32 and the low-temperature portion of the reacted gas 32 flow in opposite directions across the second partition plate 73. The second partition plate 73 can also be said to be a part of the flow path forming section 70.

In a case where the reaction device 100 includes the plurality of flow path forming sections 70 separated by the second partition plate 73, the heat exchange between the high-temperature portion of the reacted gas 32 and the low-temperature portion of the reacted gas 32 in the space 7 increases. This further improves the thermal efficiency.

The catalyst layer 3 may also include a first partition plate 33 that divides the catalyst layer 3 into a plurality of layers. As illustrated in Fig. 2, the first partition plate 33 may be a circular plate-like member that has a main surface perpendicular to the direction in which the raw material gas 31 is supplied into the catalyst layer 3. The first partition plate 33 may be located at the same height as the disk 713 of the baffle 71. An outer edge (outer side surface) of the first partition plate 33 may also be joined to the inner side surface of the disk 713 of the baffle 71. As illustrated in Fig. 2, the first partition plate 33 is located so as to divide each opening 61 in the dividing wall 60. In other words, the opening 61 is located on an upstream side and a downstream side of the first partition plate 33.

Alternatively, in a case where the catalyst layer 3 includes the first partition plate 33, the dividing wall 60 may have an inflow opening on an upstream side of the first partition plate 33 and an outflow opening on a downstream side of the first partition plate 33, separately as openings 61. The inflow opening is located at a height position that is from an upper surface of the first partition plate 33 to an upper end of the baffle 71. The outflow opening is located at a height position that is from a lower surface of the first partition plate 33 to a lower end of the baffle 71. The first partition plate 33 may be made of a material that is difficult for the reacted gas 32 to pass through. The first partition plate 33 may be made of, for example, carbon steel or stainless steel such as SUS304.

In a case where the catalyst layer 3 includes the first partition plate 33, the reacted gas 32 is efficiently guided to the space 7 through an opening 61. In other words, the first partition plate 33 can assist the reacted gas 32 to flow into the flow path formed by the flow path forming section 70. The catalyst layer 3 may include a plurality of first partition plates 33 depending on the number of flow path forming sections 70.

### (Chemical reaction method using reaction device 100)

With use of the reaction device 100 described above, a chemical reaction method in accordance with an embodiment of the present invention is carried out as follows.

First, a raw material gas 31 is supplied to the catalyst layer 3 (supply step). The supplied raw material gas 31 is brought into contact with a catalyst 30 in the catalyst layer 3, so that a reaction proceeds. This causes a reacted gas 32 to be produced in the catalyst layer 3. At least part of the reacted gas 32 produced in the catalyst layer 3 flows into the space 7 through an opening 61 in the dividing wall 60.

The reacted gas 32 that has flowed from the catalyst layer 3 into the flow path in the space 7 formed by the flow path forming section 70 is pre-cooled by heat exchange via the baffle 71 before the reacted gas 32 reaches the cooling surface 53 of the first heat exchange section 52 (pre-cooling step).

The pre-cooled reacted gas 32 is cooled on the cooling surface 53 of the first heat exchange section 52 to be separated into a condensed product and a remaining reacted gas 32 (separation step).

The remaining reacted gas 32 thus separated is preheated by heat exchange through the baffle 71 before being returned to the catalyst layer 3 (preheating step). The reacted gas 32 thus preheated is supplied to the catalyst layer 3 again through an opening 61 (resupply step).

As described above, the chemical reaction method in accordance with an embodiment of the present invention includes: the pre-cooling step of decreasing the temperature of the reacted gas 32 before the reacted gas 32 is cooled on the cooling surface 53 to a temperature at which the reacted gas 32 can be condensed; and the preheating step of increasing the temperature of the reacted gas 32 before the reacted gas undergoes a reaction in the catalyst layer 3. This makes it possible to improve the thermal efficiency of the entire reaction carried out using the reaction device 100.

### (Variation of flow path forming section)

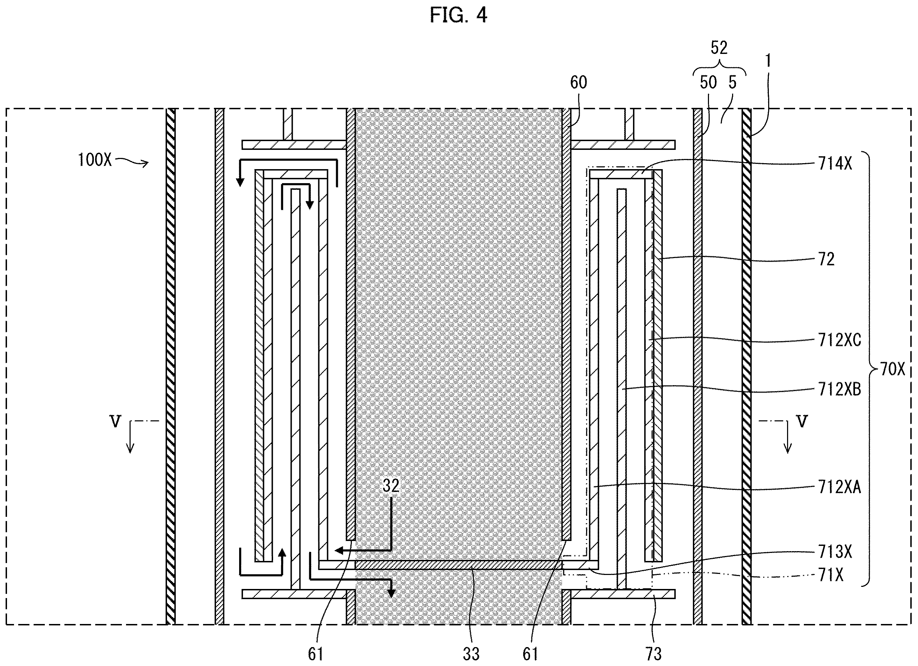

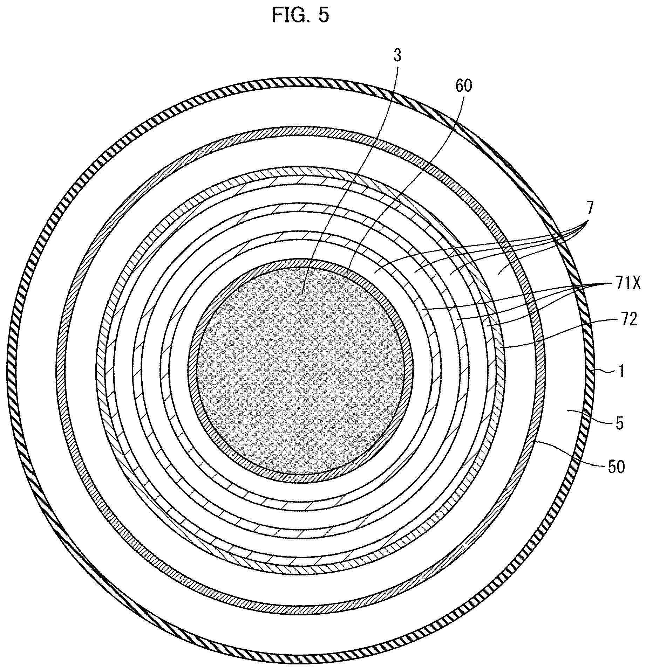

Fig. 4 is a partially enlarged view of a cross section of a reaction device 100X including a flow path forming section 70X, the cross section being obtained when the reaction device 100X is cut along a plane perpendicular to a bottom surface. Fig. 5 is a cross-sectional view taken along line V-V in Fig. 4. The flow path forming section 70X includes a baffle 71X. The baffle 71X includes three cylinders 712XA, 712XB, and 712XC which extend in the Z-axis direction and have respective different inner diameters. The inner diameters of the three cylinders 712XA, 712XB, and 712XC increase in this order. The baffle 71X also includes two disks 713X and 714X. The reaction device 100 and the reaction device 100X are similar in configuration, except for the inclusion of the flow path forming section 70X instead of the flow path forming section 70.

A distance from an inner side of the cylinder 712XA to an outer side of the cylinder 712XC may be in a range of 1/15 to 1/1.2 of a distance between a dividing wall 60 and a first heat exchange section 52. A plate thickness of each of the cylinders 712XA, 712XB, and 712XC may be in a range of 1/60 to 1/4.5 of the distance between the dividing wall 60 and the first heat exchange section 52. A plate thickness of each of the disks 713X and 714X is not particularly specified, but may be similar to the plate thickness of each of the cylinders 712XA, 712XB, and 712XC.

An inner edge (inner side surface) of the disk 713X is joined to a portion of the dividing wall 60 which portion is located between a plurality of openings 61 provided at a given height position. Alternatively, the inner edge of the disk 713X may be joined to a first partition plate 33.

An outer end part of the disk 713X is joined to a lower end of the cylinder 712XA. An upper end of the cylinder 712XA is joined to an inner end part of the disk 714X. An outer end part of the disk 714X is joined to an upper end of the cylinder 712XC. The upper ends of the cylinders 712XA and 712XC are located at approximately the same height. An upper end of the cylinder 712XB is located lower than the upper ends of the cylinders 712XA and 712XC. A lower end of the cylinder 712XB is joined to an upper surface of a second partition plate 73.

By having the above configuration, the flow path forming section 70X forms a flow path for a reacted gas 32 that has flowed from a catalyst layer 3 into a space 7. The baffle 71X enables heat exchange between parts of the reacted gas 32 which parts flow in opposite directions with the cylinders 712XA, 712XB, and 712XC interposed therebetween in the flow path of the reacted gas 32 in the space 7.

By the above heat exchange, a high-temperature portion of the reacted gas 32, which has flowed into the space 7 from the catalyst layer 3, travels toward the first heat exchange section 52 while being cooled by the heat exchange. That is, after flowing into the space 7, the high-temperature portion of the reacted gas 32 is pre-cooled and travels toward the first heat exchange section 52. The low-temperature portion of the reacted gas 32, which has been cooled by the first heat exchange section 52, travels toward the catalyst layer 3 while being heated by heat exchange. That is, after being cooled at the first heat exchange section 52, the low-temperature portion of the reacted gas 32 is preheated and returns to the catalyst layer 3. The heat exchange thus carried out in the space 7 improves the thermal efficiency of the reaction device 100.

The flow path forming section 70X may include a heat insulating member 72 located between the baffle 71X and the first heat exchange section 52. For example, as illustrated in Fig. 4, the heat insulating member 72 may be provided on an outer surface of the cylinder 712XC of the baffle 71X. Alternatively, the heat insulating member 72 may be located between a part of the flow path at the flow path forming section 70X and the first heat exchange section 52. For example, the heat insulating member 72 may be provided on an outer surface of the cylinder 712XB of the baffle 71X. This configuration makes it possible to reduce an effect of heat from the first heat exchange section 52 on the baffle 71X and further improve the efficiency of heat exchange by the baffle 71X.

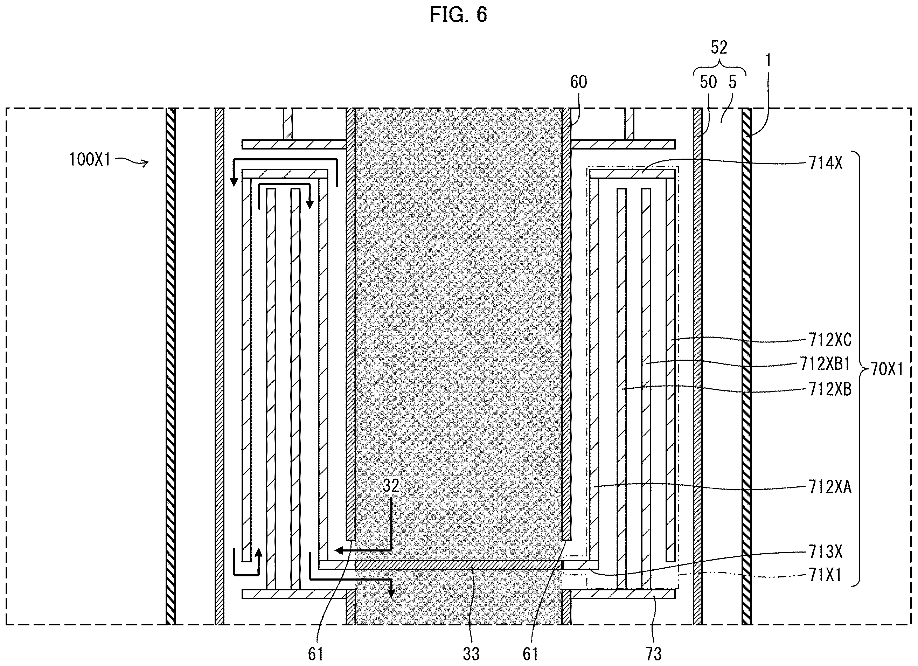

Fig. 6 is a cross-sectional view of a main part of a reaction device 100X1, which is a variation of the reaction device 100X. The reaction device 100X1 includes a flow path forming section 70X1. The flow path forming section 70X1 includes a baffle 71X1. The baffle 71X1 differs from the baffle 71X illustrated in Fig. 4 in that a cylinder 712XB1 is provided between a cylinder 712XB and a cylinder 712XC. A lower end of the cylinder 712XB1 is joined to an upper surface of a second partition plate 73, similarly as the cylinder 712XB. A space between the cylinder 712XB and the cylinder 712XB1 is a closed space, in which gas stays. This structure formed by of the cylinder 712XB, the cylinder 712XB1, and the space between the two cylinders functions as a heat insulating structure.

At the baffle 71X1, a high-temperature portion of the reacted gas 32, which has flowed into a space 7 from a catalyst layer 3, travels toward a first heat exchange section 52 while being cooled by heat exchange through a cylinder 712XA. A low-temperature portion of the reacted gas 32, which has been cooled by the first heat exchange section 52, travels toward the catalyst layer 3 while being heated by heat exchange through the cylinder 712XA.

That is, the baffle 71X1 includes, as a heat exchange section, the cylinder 712XA which enables heat exchange between a portion of the reacted gas 32 which portion flows from the catalyst layer 3 to the first heat exchange section 52 and a portion of the reacted gas 32 which portion flows from the first heat exchange section 52 to the catalyst layer 3. Further, the baffle 71X1 has a heat insulating structure formed by the cylinder 712XB, the cylinder 712XB1, and the space between the two cylinders. This configuration makes it possible to perform heat exchange while reducing the effect of heat from the first heat exchange section 52 on the heat exchange section, without providing an additional heat insulating member.

### Embodiment 2

The following description will discuss another embodiment of the present invention. For convenience of explanation, the same reference signs will be given to members each having the same function as a member described in the above embodiment, and descriptions on such a member will be omitted.

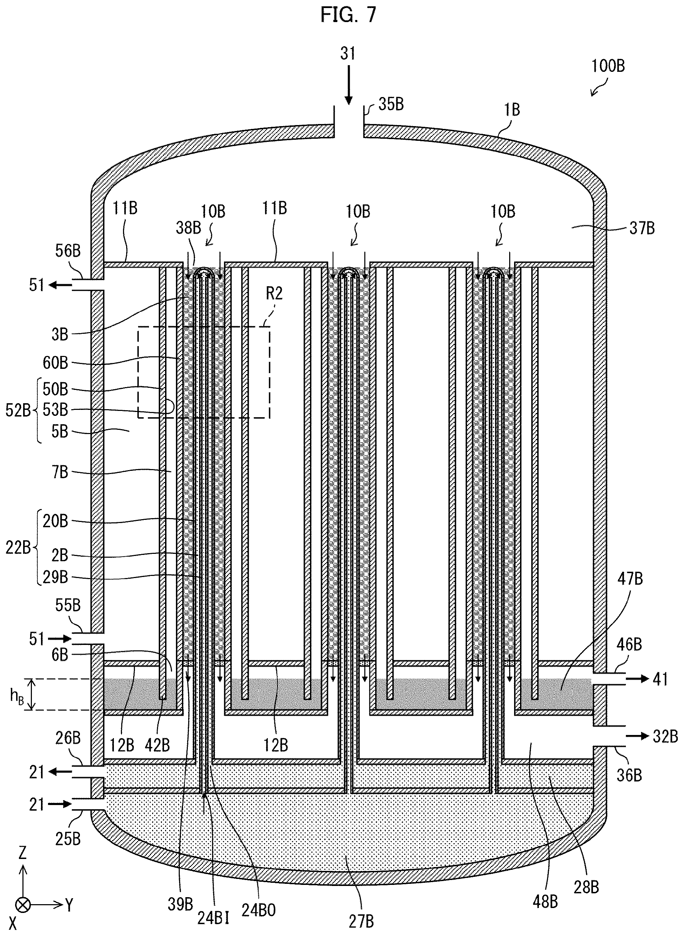

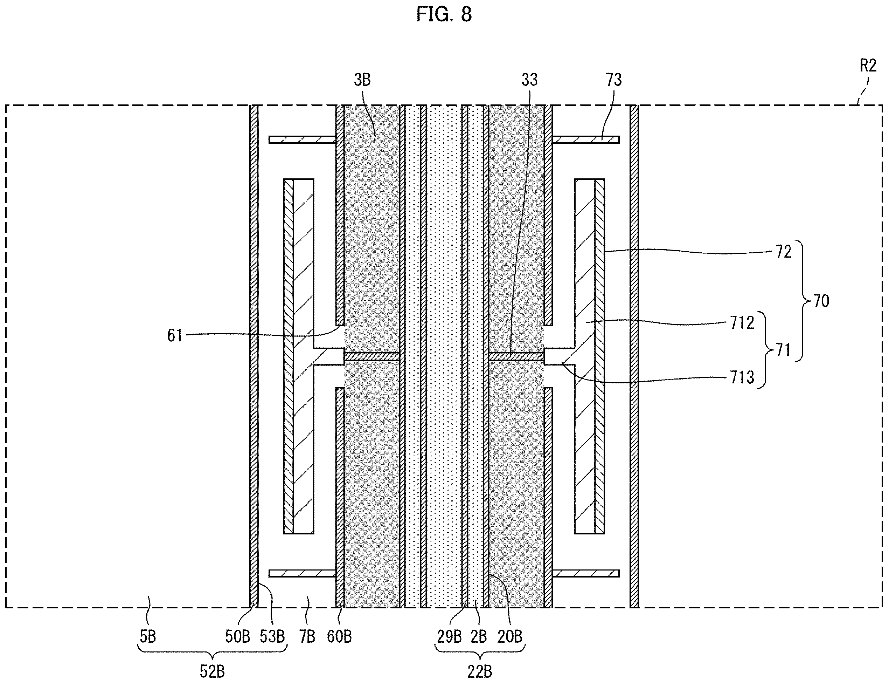

Fig. 7 is a cross-sectional view of the reaction device 100B in accordance with Embodiment 2, the cross-sectional view being taken along a plane perpendicular to a bottom surface of the reaction device 100B. Fig. 8 is an enlarged view of a region R2 in Fig. 7. Basic principles of the reaction device 100B are the same as those of the reaction device 100. The reaction device 100B includes a reaction container 1B and a plurality of reaction tubes 10B provided inside the reaction container 1B. The reaction device 100B also includes a catalyst layer 3B provided in each of the plurality of reaction tubes 10B and a second heat exchange section 22B provided at a center part of the catalyst layer 3B.

The number of the reaction tubes 10B provided inside the reaction container 1B is not particularly limited, provided that the number is at least one. In consideration of reaction efficiency, the number of the reaction tubes 10B is preferably more than one. On an upper side of the plurality of reaction tubes 10B, a raw material gas supplying section 37B is provided which is filled with a raw material gas 31 to be supplied to each of the reaction tubes 10B. On a lower side of the plurality of reaction tubes 10B, a condensate storing section 47B (liquid storing section) is provided to store therein a liquid that has been condensed inside each of the plurality of reaction tubes 10B. On a lower side of the condensate storing section 47B, a gas collecting section 48B is provided which stores therein gas that has passed through the catalyst layer 3B. On a lower side of the gas collecting section 48B, a second heat transfer medium collecting section 28B is provided which stores therein a second heat transfer medium 21 discharged from the second heat exchange section 22B (described later). Further, on a lower side of the heat transfer medium collecting section 28B, a second heat transfer medium supplying section 27B is provided which stores therein the second heat transfer medium 21 to be supplied to the second heat exchange section 22B. The above sections are formed by using a metal plate (e.g. a plate made of carbon steel or stainless steel such as SUS304) to partition an inner space of the reaction container 1B.

Each reaction tube 10B includes, in this order from an outer side, an outer cylinder 50B, a dividing wall 60B spaced apart via a space 7B from an inner wall surface of the outer cylinder 50B, the catalyst layer 3B which has a cylindrical shape and is in contact with an inner wall surface of the dividing wall 60B, and the second heat exchange section 22B provided inside the catalyst layer 3B.

The catalyst layer 3B is a region which is filled with the catalyst 30 that promotes a reaction and in which, upon supply of the raw material gas 31 into the catalyst layer 3B, the raw material gas 31 and the catalyst 30 come into contact with each other, so that a reaction proceeds. The catalyst layer 3B is filled with the catalyst 30 which is appropriate for the reaction. A portion of an upper end of the reaction tube 10B other than an upper end of the catalyst layer 3B is covered with a metal cap so that it is impossible for gas to flow through the portion. An opening 38B is formed at the upper end of the catalyst layer 3B, and the raw material gas 31 is supplied to the catalyst layer 3B through the opening 38B. At a lower end of the catalyst layer 3B, an opening 39B is formed. The opening 39B is provided with a supporting member made of, for example, a metal mesh. The supporting member serves to prevent the catalyst 30 from falling down. A first partition plate 33 located in the catalyst layer 3B may be as described in Embodiment 1.

A first heat exchange section 52B is a heat exchanger whose surface temperature on a space 7B side is maintained not higher than a dew point of the reacted gas 32. The first heat exchange section 52B is spaced apart via the space 7B from the catalyst layer 3B. The first heat exchange section 52B is constituted by: a part of an inner wall surface of the reaction container 1B; an outer wall surface of the outer cylinder 50B; and metal plates 11B and 12B. Inside the first heat exchange section 52B, a first heat transfer medium region 5B, which is common to the plurality of reaction tubes 10B, is provided. In the first heat transfer medium region 5B, a first heat transfer medium 51 is caused to flow. The outer cylinder 50B has a cooling surface 53B on a space 7B side.

A side wall of the reaction container 1B has formed therein (i) a first heat transfer medium feed opening 55B for supplying the first heat transfer medium 51 to the first heat transfer medium region 5B and (ii) a first heat transfer medium collection opening 56B for discharging the first heat transfer medium 51 from the first heat transfer medium region 5B. The first heat exchange section 52B can maintain a temperature of the outer cylinder 50B not higher than a dew point of a reacted gas 32 by letting the first heat transfer medium 51 flow through the first heat transfer medium region 5B.

The second heat exchange section 22B is a heat exchanger whose surface temperature on a catalyst layer 3B side is maintained higher than a dew point of the reacted gas 32. More specifically, as illustrated in Figs. 7 and 8, the second heat exchange section 22B includes a second heat exchange wall 20B and an inner tube 29B. Between the second heat exchange wall 20B and the inner tube 29B, a second heat transfer medium region 2B is formed. The second heat transfer medium region 2B is a region through which the second heat transfer medium 21 is caused to flow. In Embodiment 2, the second heat exchange section 22B is located on an inner side of the catalyst layer 3B.

The second heat exchange wall 20B more preferably has a temperature that enables a temperature of the entire catalyst layer 3B to be maintained higher than the dew point of the reacted gas 32. Examples of the second heat transfer medium 21 in a case where the product is methanol include high-pressure boiler water (e.g. saturated water at 2.2 MPaG to 5.0 MPaG) at 220°C to 265°C, a molten metal salt (e.g. a mixture of sodium nitrite and potassium nitrate), and heat transfer oil. Note that numerical expressions such as "A to B" herein mean "not less than A and not more than B".

The second heat exchange section 22B can maintain a temperature of the second heat exchange wall 20B higher than the dew point of the reacted gas 32 by letting the second heat transfer medium 21 flow through the second heat transfer medium region 2B. In a case where a reaction that occurs in the catalyst layer 3 is an exothermic reaction, the second heat transfer medium 21 serves as a heating medium for cooling reaction heat generated by the reaction. In a case where a reaction that occurs in the catalyst layer 3B is an endothermic reaction, the second heat transfer medium 21 serves as a heat transfer medium for heating the catalyst layer 3B. This allows the second heat transfer medium 21 to maintain a temperature of the catalyst layer 3B not less than the dew point of the reacted gas 32 and thus makes it possible to reduce the likelihood that the reacted gas 32 is condensed in the catalyst layer 3B.

The second heat exchange section 22B has a double cylinder structure, and a tip of the inner tube 29B is connected to an inflow port 24BI of the second heat transfer medium supplying section 27B. The second heat transfer medium 21 inside the second heat transfer medium supplying section 27B is supplied into the second heat exchange section 22B through the inner tube 29B. A channel between an outer wall surface of the inner tube 29B and an inner wall surface of the second heat exchange wall 20B communicates with an inside of the second heat transfer medium collecting section 28B. The second heat transfer medium 21 that has passed through an inside of the inner tube 29B passes through the channel, and is discharged to the second heat transfer medium collecting section 28B through an outflow port 24BO formed in an upper wall surface of the second heat transfer medium collecting section 28B.

The dividing wall 60B is located between the catalyst layer 3B and the space 7B and has an opening 61 through which the reacted gas 32 passes. Configurations of the flow path forming section 70 and the second partition plate 73 located in the space 7B may be as described in Embodiment 1.

The space 7B is formed between the dividing wall 60B and the outer cylinder 50B. In the present embodiment, the condensate storing section 47B is provided on a vertically lower side of the space 7B. Further, a condensate flow tube 42B (extension tube) is provided inside the condensate storing section 47B so as to extend the outer cylinder 50B vertically downward. A lower end of the condensate flow tube 42B is positioned so as to be immersed in a condensate 41 stored in the condensate storing section 47B. The condensate flow tube 42B forms, inside the condensate storing section 47B, a space (second space) 6B that is continuous with the space 7B.

A liquid (condensate 41) that has been condensed in the space 7B passes through the space 6B on an inner side of the condensate flow tube 42B and is stored in the condensate storing section 47B. The condensate 41 stored in the condensate storing section 47B is collected through a condensate collection opening 46B. At this time, discharge of fluid through the condensate collection opening 46B is controlled to prevent the reacted gas 32 from being collected through the condensate collection opening 46B.

On a vertically lower side of the catalyst layer 3B is the gas collecting section 48B (gas storing section) into which gas that has passed through the catalyst layer 3B flows. The gas collecting section 48B has an uncondensed gas collection opening 36B (exhaust section).

Thus, the condensate storing section 47B and the gas collecting section 48B form respective different spaces. The condensate storing section 47B forms a closed space with respect to gas. As such, the raw material gas 31 that has descended in the space 7B has no path forward and thus returns to the catalyst layer 3B. This makes it possible to reduce the likelihood that the raw material gas 31 passing through the space 7B proceeds to be discharged to an outside.

The second heat transfer medium supplying section 22B penetrates through the gas collecting section 48B, and a lower end of the inner tube 29B of the second heat exchange section 22B is open to an inner space of the second heat transfer medium supplying section 27B. A channel between an outer wall surface of the inner tube 29B and an inner wall surface of the second heat exchange wall 20B communicates with an inside of the second heat transfer medium collecting section 28B. The second heat transfer medium 21 that has reached an upper end of the inner tube 29B after exiting the second heat transfer medium supplying section 27B passes through the channel and is discharged to the second heat transfer medium collecting section 28B through an outflow port 24BO formed in an upper wall surface of the second heat transfer medium collecting section 28B.

The reaction device 100B in accordance with Embodiment 2 is a chemical reaction device that causes a reaction to proceed, wherein a product of the reaction contains a component having a boiling point higher than that of the raw material gas 31, and progress of the reaction in a gaseous phase is restricted by a chemical equilibrium between a raw material and the product. The reaction device 100B includes: a catalyst layer 3B to which the raw material gas 31 is supplied and which is filled with a catalyst 30 that promotes the reaction; a first heat exchange section 52B provided apart from the catalyst layer 3B with a space 7B between the first heat exchange section 52B and the catalyst layer 3B, a temperature of a surface of the first heat exchange section 52B on a space 7B side being maintained not higher than a dew point of a reacted gas 32 produced by the reaction; a dividing wall 60B located between the catalyst layer 3B and the space 7B and having an opening 61 through which the reacted gas 32 passes; and a flow path forming section 70 located within the space 7B and forming a flow path for the reacted gas 32 that flows from the catalyst layer 3B into the space 7B through the opening 61. The flow path forming section 70 includes the baffle 71 which enables heat exchange between a portion of the reacted gas 32 which portion flows from the catalyst layer 3B toward the first heat exchange section 52B and a portion of the reacted gas 32 which portion flows from the first heat exchange section 52B toward the catalyst layer 3B.

This configuration makes it possible to improve the thermal efficiency of the reaction carried out using the reaction device 100B.

### Embodiment 3

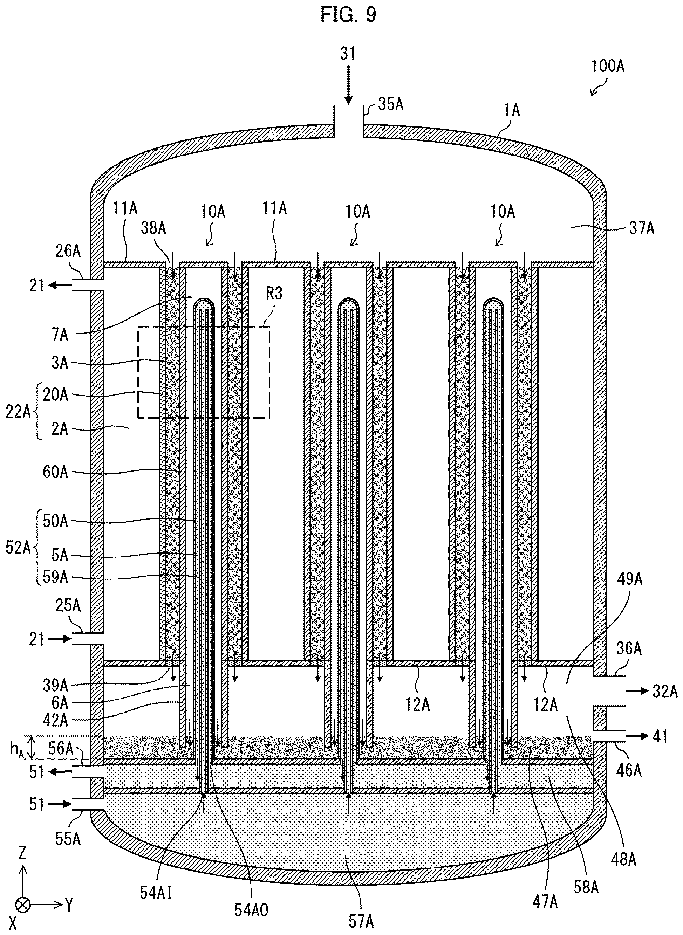

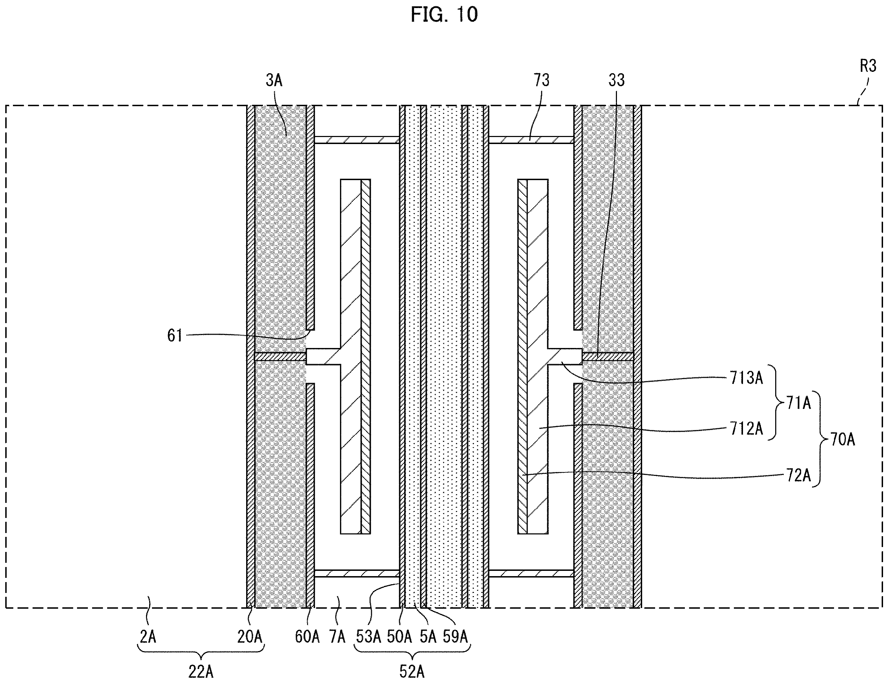

Fig. 9 is a cross-sectional view of a reaction device 100A in accordance with Embodiment 3, the cross-sectional view being taken along a plane perpendicular to a bottom surface of the reaction device 100A. Fig. 10 is an enlarged view of a region R3 in Fig. 9. As illustrated in Fig. 9, the reaction device 100A includes a reaction container 1A and a plurality of reaction tubes 10A provided inside the reaction container 1A. The reaction device 100A differs from Embodiment 1 and Embodiment 2 in that a space 7A is located on an inner side with respect to a catalyst layer 3A.

On an upper side of the plurality of reaction tubes 10A, a raw material gas supplying section 37A is provided which is filled with a raw material gas 31 to be supplied to each of the reaction tubes 10A. On a lower side of the plurality of reaction tubes 10A, a storing section 48A is provided which stores therein a liquid that has been condensed inside each reaction tube 10A and gas that has passed through a catalyst layer 3A. On a lower side of the storing section 48A, a first heat transfer medium collecting section 58A is provided which stores therein a first heat transfer medium 51 discharged from a first heat exchange section 52A (described later). Further, on a lower side of the first heat transfer medium collecting section 58A, a first heat transfer medium supplying section 57A is provided which stores therein a first heat transfer medium 51 to be supplied to the first heat exchange section 52A. The above sections are formed by using a metal plate (e.g. a plate made of carbon steel or stainless steel such as SUS304) to partition an inner space of the reaction container 1A.

The reaction tube 10A is open to a metal plate 11A located in an upper part of the reaction tube 10A and to a metal plate 12A located in a lower part of the reaction tube 10A. An outer cylinder 20A of the reaction tube 10A is joined to the metal plates 11A and 12A by welding.

As illustrated in Figs. 9 and 10, the reaction tube 10A includes, in this order from an outer side, the outer cylinder 20A, the catalyst layer 3A which has a cylindrical shape and is in contact with an inner wall surface of the outer cylinder 20A, a dividing wall 60A provided on an inner side of the catalyst layer 3A, and the first heat exchange section 52A. The first heat exchange section 52A is spaced apart via the space 7A (first space) from the dividing wall 60A. The dividing wall 60A is located between the catalyst layer 3A and the space 7A and has an opening 61 through which a reacted gas 32 passes.

The catalyst layer 3A and the catalyst layer 3B described above in Embodiment 2 are similar in configuration.

The first heat exchange section 52A is a heat exchanger whose surface temperature on a space 7A side is maintained not higher than a dew point of the reacted gas 32. The first heat exchange section 52A is spaced apart via the space 7A from the catalyst layer 3A and has a cooling surface 53A on the space 7A side.

As illustrated in Figs. 9 and 10, the first heat exchange section 52A includes a first heat exchange wall 50A and an inner tube 59A. Between the first heat exchange wall 50A and the inner tube 59A, a first heat transfer medium region 5A is formed. The first heat transfer medium region 5A is a region through which the first heat transfer medium 51 flows.

The first heat exchange section 52A has a double cylinder structure, and a tip of the inner tube 59A is connected to an inflow port 54I of the first heat transfer medium supplying section 57A. The first heat transfer medium 51 inside the first heat transfer medium supplying section 57A is supplied into the first heat exchange section 52A through the inner tube 59A. A channel between an outer wall surface of the inner tube 59A and an inner wall surface of the first heat exchange wall 50A communicates with an inside of the first heat transfer medium collecting section 58A. The first heat transfer medium 51 that has reached an upper end of the inner tube 59A after exiting the first heat transfer medium supplying section 57A passes through the channel and is discharged to the first heat transfer medium collecting section 58A through an outflow port 54AO formed in an upper wall surface of a first heat transfer medium collecting section 58A.

The first heat exchange wall 50A is made of a member that does not allow fluids to pass therethrough. A surface of the first heat exchange wall 50A on a space 7A side serves as a first heat exchange surface (cooling surface). The first heat exchange section 52A can maintain a temperature of the first heat exchange wall 50A not higher than the dew point of the reacted gas 32 by letting the first heat transfer medium 51 flow through the first heat transfer medium region 5A.

A second heat exchange section 22A is a heat exchanger whose surface temperature on a catalyst layer 3A side is maintained higher than the dew point of the reacted gas 32. The second heat exchange section 22A is constituted by: a part of an inner wall surface of the reaction container 1A; an outer wall surface of the outer cylinder 20A; and the metal plates 11A and 12A. Inside the second heat exchange section 22A, a second heat transfer medium region 2A, which is common to the plurality of reaction tubes 10A, is provided. In the second heat transfer medium region 2A, a second heat transfer medium 21 is caused to flow. The outer cylinder 20A is made of a member that does not allow fluids to pass therethrough. A surface of the outer cylinder 20A on the catalyst layer 3A side serves as a second heat exchange surface.

A side wall of the reaction container 1A has formed therein (i) a second heat transfer medium feed opening 25A for supplying the second heat transfer medium 21 to the second heat transfer medium region 2A and (ii) a second heat transfer medium collection opening 26A for discharging the second heat transfer medium 21 from the second heat transfer medium region 2A. The second heat exchange section 22A can maintain a temperature of the outer cylinder 20A higher than the dew point of the reacted gas 32 by letting the second heat transfer medium 21 to flow through the second heat transfer medium region 2A.

The dividing wall 60A is located between the catalyst layer 3A and the space 7A and has an opening 61 through which the reacted gas 32 passes. A flow path forming section 70A is located within the space 7 and includes a baffle 71A.

In the present embodiment, the baffle 71A includes a cylinder 712A which extends in the Z-axis direction and a disk 713A which is joined to an outer wall of the cylinder 712. An inner diameter of the cylinder 712A is more than an outer diameter of the first heat exchange section 52A (first heat exchange wall 50A), and an outer diameter of the cylinder 712A is less than an inner diameter of the dividing wall 60A. The cylinder 712A may have a thickness in a range of 1/15 to 1/1.2 of a distance between the dividing wall 60A and the first heat exchange section 52A.

The disk 713A is a plate-like member having a doughnut shape and connects the outer wall of the cylinder 712A to the dividing wall 60A (or to a first partition plate 33, which will be described later). Specifically, an inner edge (inner side surface) of the disk 713A is connected to the cylinder 712A, and an outer edge (outer side surface) of the disk 713A is joined to a portion of the dividing wall 60A which portion is located between the plurality of openings 61 provided at a given height position. Alternatively, the outer edge of the disk 713A may be joined to the first partition plate 33. The baffle 71A is made of a material similar to that of the baffle 71 in accordance with Embodiment 1. The baffle 71A enables heat exchange between a high-temperature portion of the reacted gas 32, which has been heated by a reaction heat of a reaction in the catalyst layer 3A, and a low-temperature portion of the reacted gas 32, which has been cooled by the first heat exchange section 52A.

The flow path forming section 70A may include a heat insulating member 72A located between the baffle 71A and the first heat exchange section 52A. For example, as illustrated in Fig. 10, the heat insulating member 72A may be provided on a surface of the baffle 71A which surface faces the first heat exchange section 52A. Configurations of the flow path forming section 70A and a second partition plate 73 located in the space 7A may be as described in Embodiment 1.

The space 7A is formed between the dividing wall 60A and the first heat exchange wall 50A. In the present embodiment, a condensate flow tube (communicating tube) 42A is provided on a vertically lower side of the space 7A. The condensate flow tube 42A is formed so that the space 7A is extended vertically downward. The condensate flow tube 42A is made of a member that does not allow a liquid to pass therethrough. More specifically, the condensate flow tube 42A forms, between itself and the surface of the first heat exchange section 52A, a space 6A that is continuous with the space 7A.

On a lower side of the reaction tube 10A, the storing section 48A is provided which stores therein, on a vertically lower side of the space 7A, a condensate 41 that has been produced in the space 7A and gas that has passed through the catalyst layer 3A. The condensate flow tube 42A is provided inside the storing section 48A, and a lower end of the condensate flow tube 42A is positioned so as to be immersed in the condensate 41 stored in a bottom part (referred to as a "condensate storing section 47A") of the storing section 48A.

In a space (referred to as a "gas collection region 49A") on an upper side inside the storing section 48A, an uncondensed gas 32A that has passed through the catalyst layer 3A is stored. The uncondensed gas 32A is a portion of the reacted gas 32 which portion passes through the catalyst layer 3A without being condensed, flows in the gas collection region 49A (storing section 48A), comes into contact with the condensate storing section (liquid storing section) 47A, and then is discharged. The storing section 48A has an uncondensed gas collection opening 36A (exhaust section) through which the uncondensed gas 32A stored in the gas collection region 49A is discharged. The uncondensed gas collection opening 36A is located vertically above the lower end of the condensate flow tube 42A.

The condensate 41, which is a product that has been condensed in the space 7A, passes through an inside of the condensate flow tube 42A and is discharged to the condensate storing section 47A. The condensate 41 in the condensate storing section 47A is collected through a condensate collection opening 46A that is provided in the vicinity of a bottom part of the storing section 48A. At this time, discharge of a fluid through the condensate collection opening 46A is controlled to prevent the reacted gas 32 from being collected through the condensate collection opening 46A.

The reaction device 100A in accordance with Embodiment 3 is a chemical reaction device that causes a reaction to proceed, wherein a product of the reaction contains a component having a boiling point higher than that of the raw material gas 31, and progress of the reaction in a gaseous phase is restricted by a chemical equilibrium between a raw material and the product. The reaction device 100A includes: the catalyst layer 3A to which the raw material gas 31 is supplied and which is filled with the catalyst 30 that promotes the reaction; the first heat exchange section 52A provided apart from the catalyst layer 3A with the space 7A between the first heat exchange section 52A and the catalyst layer 3A, the temperature of the surface of the first heat exchange section 52A on the space 7A side being maintained not higher than a dew point of a reacted gas 32 produced by the reaction; the dividing wall 60A located between the catalyst layer 3A and the space 7A and having an opening 61 through which the reacted gas 32 passes; and the flow path forming section 70A located within the space 7A and forming a flow path for the reacted gas 32 that flows from the catalyst layer 3A into the space 7A through the opening 61. The flow path forming section 70A includes the baffle 71A which enables heat exchange between a portion of the reacted gas 32 which portion flows from the catalyst layer 3A toward the first heat exchange section 52A and a portion of the reacted gas 32 which portion flows from the first heat exchange section 52A toward the catalyst layer 3A.

This configuration makes it possible to improve the thermal efficiency of the reaction carried out using the reaction device 100A.

Aspects of the present invention can also be expressed as follows:
(1) A chemical reaction device in accordance with Aspect 1 of the present disclosure is a chemical reaction device that causes a reaction to proceed, a product of the reaction containing a component having a boiling point higher than that of a raw material gas, progress of the reaction in a gaseous phase being restricted by a chemical equilibrium between a raw material and the product, the chemical reaction device including: a catalyst layer to which the raw material gas is supplied and which is filled with a catalyst that promotes the reaction; a first heat exchange section provided apart from the catalyst layer with a space between the first heat exchange section and the catalyst layer, a temperature of a surface of the first heat exchange section on a space side being maintained not higher than a dew point of a reacted gas produced by the reaction; a dividing wall located between the catalyst layer and the space and having an opening through which the reacted gas passes; and a flow path forming section located within the space and forming a flow path for the reacted gas that flows from the catalyst layer into the space through the opening, the flow path forming section includes a baffle that enables heat exchange in the flow path between a portion of the reacted gas which portion flows from the catalyst layer toward the first heat exchange section and a portion of the reacted gas which portion flows from the first heat exchange section toward the catalyst layer.
   With the above configuration, heat exchange is carried out in the space, between a portion of the reacted gas which portion flows from the catalyst layer toward the first heat exchange section and a portion of the reacted gas which portion flows from the first heat exchange section toward the catalyst layer. This makes it possible to improve the thermal efficiency.
(2) A chemical reaction device in accordance with Aspect 2 of the present disclosure is configured such that, in Aspect 1, the chemical reaction device further includes a heat insulating member located between the baffle and the first heat exchange section.
   The above configuration makes it possible to reduce an effect of heat from the first heat exchange section on the baffle. This makes it possible to further improve the efficiency of heat exchange by the baffle and improve the thermal efficiency.
(3) A chemical reaction device in accordance with Aspect 3 of the present disclosure is configured such that, in Aspect 1 or 2, the chemical reaction device further includes: a first partition plate that is provided in the catalyst layer, that has a main surface perpendicular to a direction in which the raw material gas is supplied, and that divides the catalyst layer into a plurality of layers, the opening being located on an upstream side and a downstream side of the first partition plate.
   With the above configuration, since the catalyst layer includes the first partition plate, the reacted gas is efficiently guided to the space through the opening. This makes it possible to improve the efficiency of heat exchange in the space.
(4) A chemical reaction device in accordance with Aspect 4 of the present disclosure is configured such that, in any one of Aspects 1 to 3, (i) a plurality of flow path forming sections, each of which is the flow path forming section, are arranged along a direction in which the raw material gas is supplied, and (ii) the plurality of flow path forming sections are separated from each other by a second partition plate that enables heat exchange between a gas that passes on one side of the second partition plate and a gas that passes on the other side of the second partition plate.
   With the above configuration, the heat exchange between the high-temperature portion of the reacted gas and the low-temperature portion of the reacted gas in the space increases. This further improves the thermal efficiency.
(5) A chemical reaction device in accordance with Aspect 5 of the present disclosure is configured such that, in any one of Aspects 1 to 4, the baffle contains a material having a thermal conductivity higher than that of the dividing wall.
   The above configuration enables heat exchange between a portion of the reacted gas which portion flows from the catalyst layer toward the first heat exchange section and a portion of the reacted gas which portion flows from the first heat exchange section toward the catalyst layer.
(6) A chemical reaction device in accordance with Aspect 6 of the present disclosure is configured such that, in any one of Aspects 1 to 4, the baffle contains at least one material selected from the group consisting of copper, aluminum, and carbon.
   With the above configuration, a material having a high thermal conductivity is used in the baffle. This improves an efficiency of heat exchange between a portion of the reacted gas which portion flows from the catalyst layer toward the first heat exchange section and a portion of the reacted gas which portion flows from the first heat exchange section toward the catalyst layer.
(7) A chemical reaction device in accordance with Aspect 7 of the present disclosure is configured such that, in any one of Aspects 1 to 6, the chemical reaction device further includes: a second heat exchange section located on an opposite side from the dividing wall with the catalyst interposed between the second heat exchange section and the dividing wall, a temperature of a surface of the second heat exchange section which surface is in contact with the catalyst being maintained higher than the dew point of the reacted gas.
   The above configuration makes it possible to reduce the likelihood that the reacted gas is condensed in the catalyst layer.
(8) A chemical reaction method in accordance with Aspect 8 of the present disclosure is a chemical reaction method in which a chemical reaction device described in any one of Aspects 1 to 7 is used, the method including: a supply step of supplying the raw material gas to the catalyst layer; a pre-cooling step of pre-cooling, by heat exchange via the baffle, the reacted gas that has flowed from the catalyst layer into the flow path through the opening; a separation step of separating the reacted gas thus pre-cooled into a condensed product and a remaining reacted gas by cooling the reacted gas on the surface of the first heat exchange section; a preheating step of preheating the remaining reacted gas by heat exchange via the baffle; and a resupply step of supplying the remaining reacted gas thus preheated to the catalyst layer.
   The chemical reaction method makes it possible to improve the thermal efficiency of a reaction carried out using an internal condensation type reaction device to proceed, a product of the reaction containing a component having a boiling point higher than that of a raw material gas, progress of the reaction in a gaseous phase being restricted by a chemical equilibrium between a raw material and the product.
(9) A chemical reaction method in accordance with Aspect 9 of the present disclosure is configured such that, in Aspect 8, the reaction is an exothermic reaction.
   The chemical reaction method makes it possible to improve the thermal efficiency of an exothermic reaction carried out using an internal condensation type reaction device to proceed, a product of the exothermic reaction containing a component having a boiling point higher than that of a raw material gas, progress of the exothermic reaction in a gaseous phase being restricted by a chemical equilibrium between a raw material and the product.
(10) A chemical reaction method in accordance with Aspect 10 of the present disclosure is configured such that, in Aspect 8, the raw material gas contains carbon oxide and hydrogen, and the product is methanol.

The chemical reaction method makes it possible to improve thermal efficiency of a methanol synthesis reaction carried out with use of an internal condensation type reaction device.

### [Validation test]

### [Simulation-based validation test]

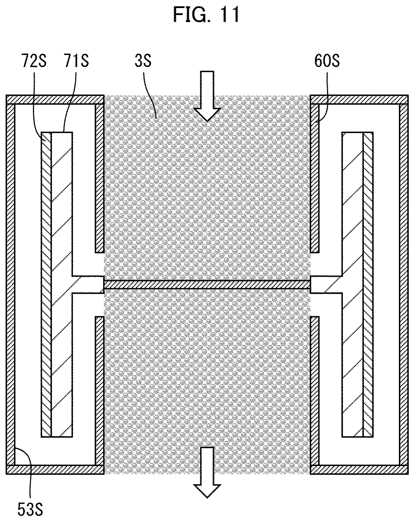

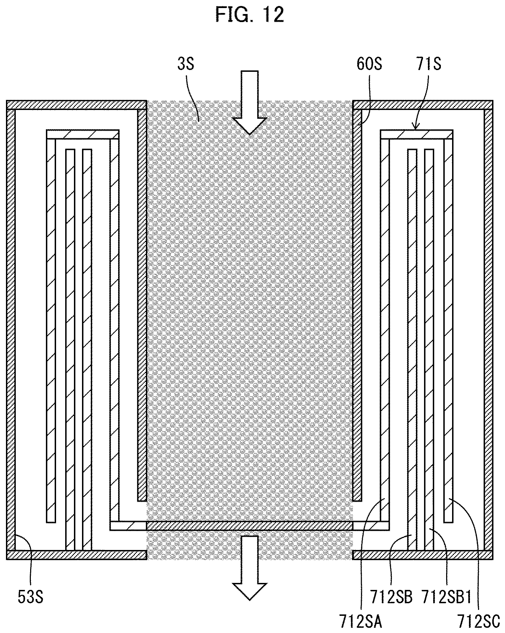

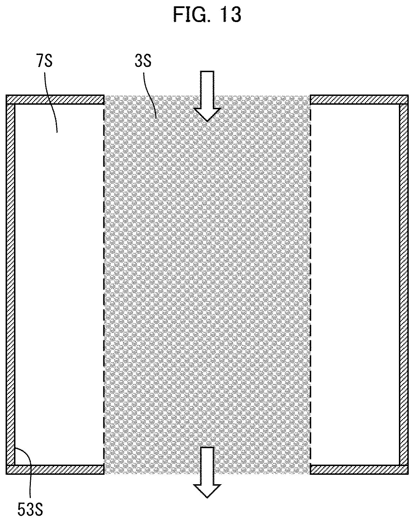

Below, results obtained through simulations are described for Example 1 and Example 2, which have configurations within the scope of the present invention, as well as for a Comparative Example, which has a configuration outside the scope of the present invention. In the simulations below, models in which no chemical reactions occur between mixed gases were adopted to reduce computational load. Similarly, to further reduce computational load, models in which phase changes such as condensation do not occur were adopted. Fig. 11 is a view schematically illustrating a configuration of a model device used for the simulation of Example 1. Fig. 12 is a view schematically illustrating a configuration of a model device used for the simulation of Example 2. Fig. 13 is a view schematically illustrating a configuration of a model device used for the simulation of the Comparative Example.

### (Example 1)

In the present simulation, a model having the configuration illustrated in Fig. 11 was used to conduct the simulation in order to verify a flow state and a temperature distribution inside a reactor in the reaction device 100 of Embodiment 1.

The simulation was performed using fluid analysis software (ANSYS Fluent 2021R2). The SST k-ω model was adopted as a turbulence model, and the SIMPLE method was used as an analysis technique. Details of the model are as described in a software manual etc.

The calculation conditions are as follows:
· With an upper face of a catalyst layer 3S in Fig. 11 serving as an inlet, 1.908 g/s of a mixed gas consisting of 1.454 g/s of CO₂ gas, 0.197 g/s of H₂ gas, and 0.257 g/s of N₂ gas is caused to flow downward. A temperature of the mixed gas at the inlet of the reactor is 220°C.
· Physical properties of the mixed gas were determined using a physical properties database registered in the fluid analysis software.
· The catalyst layer 3S has a density of 1250 kg/m³, a specific heat of 470 J/kg/K, and a thermal conductivity of 0.8 W/m/K.
· A pressure loss of the catalyst layer 3S is 80700000/m².
· Heat of 128 kW/m³ is uniformly applied to the catalyst layer 3S.
· A temperature of the cooling surface 53S is 70°C.
· A dividing wall 60S is made of SUS304L and has a density of 7930 kg/m³, a specific heat of 500 J/kg/K, and a thermal conductivity of 17.2 W/m/K.
· A baffle 71S is made of aluminum and has a density of 2729kg/m³, a specific heat of 871 J/kg/K, and a thermal conductivity of 202.4 W/m/K.
· The baffle 71S is heat-insulated on a cooling surface 53S side (where a heat insulating member 72S is located). · A pressure at an outlet of the reactor is 5 MPaG.
· In the present simulation, a model in which no chemical reactions occur between mixed gases was adopted in order to reduce computational load. Similarly, to further reduce computational load, a model in which phase changes such as condensation do not occur was adopted.

### (Example 2)

In the present simulation, a model having the configuration illustrated in Fig. 12 was used to conduct a simulation in order to verify a flow state and a temperature distribution inside a reactor in the reaction device 100X1 (Fig. 6) of Embodiment 1. The software and model used in the simulation are equivalent to those in Example 1.

The calculation conditions are as follows:
· With an upper face of a catalyst layer 3S in Fig. 12 serving as an inlet, 5.18 g/s of a mixed gas consisting of 4.17 g/s of CO₂ gas, 0.57 g/s of H₂ gas, and 0.44 g/s of N₂ gas is caused to flow downward. A temperature of the mixed gas at the inlet of the reactor is 200°C.
· Physical properties of the mixed gas were determined using the physical properties database registered in the fluid analysis software.
· The catalyst layer 3S has a density of 1250 kg/m³, a specific heat of 470 J/kg/K, and a thermal conductivity of 0.8 W/m/K.
· A pressure loss of the catalyst layer 3S is 80700000/m².
· Heat of 128 kW/m³ is uniformly applied to the catalyst layer 3S.
· A temperature of a cooling surface 53S is 70°C.
· A dividing wall 60S and a baffle 71S are each made of SUS304L and each have a density of 7930 kg/m³, a specific heat of 500 J/kg/K, and a thermal conductivity of 17.2 W/m/K.
· The baffle 71S includes a cylinder 712SA, which serves as a heat exchange section, and a heat insulating structure formed by a cylinder 712SB, a cylinder 712SB1, and a space between the two cylinders.
· A pressure at an outlet of the reactor is 5 MPaG.

### (Comparative Example)

In the present simulation, a model having the configuration illustrated in Fig. 13 was used to conduct the simulation in order to verify a flow state and a temperature distribution inside a reactor in a case where a reaction device outside the scope of the present invention is used. The model illustrated in Fig. 13 has no dividing wall between a catalyst layer 3S and a space 7S (opening ratio: 100%). Further, the model has no baffle 71S. The software and model used in the simulation are equivalent to those in Example 1.

The calculation conditions are as follows:
· With an upper face of the catalyst layer 3S in Fig. 13 serving as an inlet, 1.908 g/s of a mixed gas consisting of 1.454 g/s of CO₂ gas, 0.197 g/s of H₂ gas, and 0.257 g/s of N₂ gas is caused to flow downward. A temperature of the mixed gas at the inlet of the reactor is 220°C.
· Physical properties of the mixed gas were determined using the physical properties database registered in the fluid analysis software.
· The catalyst layer 3S has a density of 1250 kg/m³, a specific heat of 470 J/kg/K, and a thermal conductivity of 0.8 W/m/K.
· A pressure loss of the catalyst layer 3S is 80700000/m².
· Heat of 128 kW/m³ is uniformly applied to the catalyst layer 3S.
· A temperature of a cooling surface 53S is 70°C.
· A pressure at an outlet of the reactor is 5 MPaG.

The results of the simulations of Example 1, Example 2, and the Comparative Example are shown in Tables 1 and 2 below.

In the table below, (heat loss rate [-]) = (amount of heat exiting through the cooling surface [W]) / ("amount of heat of gas flowing into the reactor [W]" + "amount of heat generated in the catalyst layer [W]").

**[Table 1]**

| | Temperature at inlet of reactor [°C] | Temperature at outlet of reactor [°C] | Difference between temperature at inlet and temperature at outlet [K] |
|---|---|---|---|
| Example 1 | 220 | 206.9 | 13.1 |
| Example 2 | 200 | 186.8 | 13.2 |
| Comparative Example | 220 | 101 | 119 |

**[Table 2]**

| | Amount of heat of gas flowing into reactor [W] | Amount of heat generated in catalyst layer [W] | Amount of heat existing through cooling surface [W] | Amount of heat of gas flowing out of reactor [W] | Heat loss rate [-] |
|---|---|---|---|---|---|
| Example 1 | 872 | 492 | 552 | 808 | 0.4 |
| Example 2 | 2190 | 723 | 1124 | 1786 | 0.39 |
| Comparative Example | 1028 | 492 | 1132 | 376 | 0.74 |

As indicated in Table 2, the heat loss rate of Example 1 was 0.4, and the heat loss rate of Example 2 was 0.39. Meanwhile, the heat loss rate of the Comparative Example was 0.74. From these results, it was demonstrated that the Examples within the scope of the present invention can improve the thermal efficiency compared to the Comparative Example.

### [Supplementary note]

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

100, 100A, 100B, 100X, 100X1: Reaction device
3, 3A, 3B: Catalyst layer
7, 7, 7B: Space
52, 52A, 52B: First heat exchange section
22A, 22B: Second heat exchange section
31: Raw material gas
32: Reacted gas
33: First partition plate
60, 60A, 60B: Dividing wall
61: Opening
70, 70X, 70X1: Flow path forming section
71, 71X, 71X1: Baffle
72, 72S: Heat insulating member
73: Second partition plate

## Claims

1. A chemical reaction device that causes a reaction to proceed, a product of the reaction containing a component having a boiling point higher than that of a raw material gas, progress of the reaction in a gaseous phase being restricted by a chemical equilibrium between a raw material and the product,
the chemical reaction device comprising:
a catalyst layer to which the raw material gas is supplied and which is filled with a catalyst that promotes the reaction;
a first heat exchange section provided apart from the catalyst layer with a space between the first heat exchange section and the catalyst layer, a temperature of a surface of the first heat exchange section on a space side being maintained not higher than a dew point of a reacted gas produced by the reaction;
a dividing wall located between the catalyst layer and the space and having an opening through which the reacted gas passes; and
a flow path forming section located within the space and forming a flow path for the reacted gas that flows from the catalyst layer into the space through the opening,
the flow path forming section includes a baffle that enables heat exchange in the flow path between a portion of the reacted gas which portion flows from the catalyst layer toward the first heat exchange section and a portion of the reacted gas which portion flows from the first heat exchange section toward the catalyst layer.

2. The chemical reaction device as set forth in claim 1, further comprising:
a heat insulating member located between the baffle and the first heat exchange section.

3. The chemical reaction device as set forth in claim 1 or 2, further comprising:
a first partition plate that is provided in the catalyst layer, that has a main surface perpendicular to a direction in which the raw material gas is supplied, and that divides the catalyst layer into a plurality of layers,
the opening being located on an upstream side and a downstream side of the first partition plate.

4. The chemical reaction device as set forth in claim 1 or 2, wherein:
a plurality of flow path forming sections, each of which is the flow path forming section, are arranged along a direction in which the raw material gas is supplied; and
the plurality of flow path forming sections are separated from each other by a second partition plate that enables heat exchange between a gas that passes on one side of the second partition plate and a gas that passes on the other side of the second partition plate.

5. The chemical reaction device as set forth in claim 1 or 2, wherein the baffle contains a material having a thermal conductivity higher than that of the dividing wall.

6. The chemical reaction device as set forth in claim 1 or 2, wherein the baffle contains at least one material selected from the group consisting of copper, aluminum, and carbon.

7. The chemical reaction device as set forth in claim 1 or 2, further comprising:
a second heat exchange section located on an opposite side from the dividing wall with the catalyst interposed between the second heat exchange section and the dividing wall, a temperature of a surface of the second heat exchange section which surface is in contact with the catalyst being maintained higher than the dew point of the reacted gas.

8. A chemical reaction method in which a chemical reaction device recited in claim 1 or 2 is used, the method comprising:
a supply step of supplying the raw material gas to the catalyst layer;
a pre-cooling step of pre-cooling, by heat exchange via the baffle, the reacted gas that has flowed from the catalyst layer into the flow path through the opening;
a separation step of separating the reacted gas thus pre-cooled into a condensed product and a remaining reacted gas by cooling the reacted gas on the surface of the first heat exchange section;
a preheating step of preheating the remaining reacted gas by heat exchange via the baffle; and
a resupply step of supplying the remaining reacted gas thus preheated to the catalyst layer.

9. The chemical reaction method as set forth in claim 8, wherein the reaction is an exothermic reaction.

10. The chemical reaction method as set forth in claim 8, wherein:
the raw material gas contains carbon oxide and hydrogen; and
the product is methanol.
